# EUROPEAN PATENT APPLICATION

(11) **EP 1 040 834 A1**
(43) Date of publication of application: **04.10.2000**
(21) Application number: 98935039.2
(22) Date of filing: 29.07.1998
(51) Int. Cl.: A61K 35/78, A23L 1/29

(54) **HYDROSOLUBLE FRACTIONS OF $i(PHLEBODIUM DECUMANUM) AND USE THEREOF AS NUTRITIONAL COMPLEMENTS IN AIDS AND CANCER PATIENTS**

(30) Priority: 30.07.1997 ES 9701693; 02.06.1998 ES 9801139
(71) Applicant: Helsint S.A.L., 18005 Granada (ES); Helechos Internacional Honduras, S.A. de C.V., Tegucigalpa M.D.C. (HN)
(72) Inventor: YESARES FERRER, Miguel, E-18005 Granada (ES); MENDOZA MEDINA, Jorge, A., Tegucigalpa M.D.C. (HN); RUIZ CACERES, Giovanna, Marisol, Tegucigalpa M.D.C. (HN); ALCAIDE GARCIA, Antonio, E-28006 Madrid (ES); YESARES MORILLAS, Miguel Enrique, E-18005 Granada (ES)
(74) Representative: Patentanwälte Zellentin & Partner
(86) International application number: ES9800220
(87) International publication number: WO9906058

(57) **Abstract**

A purified and standardized water-soluble fraction, prepared from the leaves of a variety of Phlebodium decumanum and identified as EXPLY-37, is adequate for the manufacturing of formulations, useful as nutritional supplements of general application, and more particularly, in patients suffering from general weakness and cachexia, such as AIDS an cancer patients. The formulations can contain, optionally, powdered Phlebodium decumanum rhizome and/or Phlebodium decumanum rhizome extract, together with the appropriate excipients for preparing said formulation as powders, capsules and syrups.

## Description

### FIELD OF THE INVENTION

This invention relates to a purified and standardized water-soluble fraction obtained from the leaves of a cultivated variety of Phlebodium decumanum, identified as EXPLY-37, to formulations containing it and to their use as nutritional supplements of general application and particularly, in patients suffering from general weakness and cachectic syndrome, as AIDS and cancer patients. Such formulations can also be used in combination with anti-retroviral drugs in AIDS patients and with conventional anticancer treatments (surgery and / or radio - and chemotherapy ) in order to improve the efficacy and to reduce the undesired effects of such treatments. The formulations are also useful in the recovery of patients under those treatments.

### BACKGROUND OF THE INVENTION

Cachexia is a complex syndrome characterized by appetite and body weight loss, general weakness, anemia and asthenia. This syndrome is associated to a metabolic dysbalance and shown by weight loss and generalized wasting : the organism is unable to maintain the adequate energy supply and spends its own lipid store and muscle protein.

It is accepted that a high percentage ( 50-90%) of HIV infected patients suffer from some kind of malnutrition. This disturbance can be attributed to alterations in the intake, absorption and metabolism of foods [ C. Fields-Gardner, Nutr.Clin. Pract. 1.995, 10(5), 167-176.] Severe weight loss is closely related to the mortality increase in AIDS patients. It may be related, on the other hand, to some kind of gastrointestinal pathology, anorexia, systemic infections and can appear in patients with advanced disease and CD4+ count lower than 100 [D.P.Kotler, AIDS Res. Hum. Retroviruses 1.994, 10(8), 930- 934 ]

Wasting syndrome associated to HIV infection is characterized by progressive weight loss and increasing weakness, frequently associated to fever and diarrhea. The causes of these alterations are complexes, multifactorial and seem to be related to inadequate diets, malabsorption phenomena, metabolic dysbalance and activity of some cytokines such as tumor necrosis factor (TNF), IL-1, IL-6 and α- IFN [ S.E. Weinroth et al. Infect. Agents Dis. 1995, 4,76- 94] . P. Kelly et al. [ Q.S. Med. 1.996, 89 (II), 831-837 ] found a relationship between the severity of cachexia in a group of African males and the high cytokine activity, specially high concentrations of sTNF55 , although no correlation could be established with either oesophagic candidiasis or any intestinal opportunistic infection.

The relationship between wasting syndrome and low blood levels of testosterone has been demonstrated in male AIDS patients . Special diets, testosterone, nandrolone [ J. Gold et at 1.996, 10 (7), 108-112] and growth hormone [M. Shambelan et al. Ann. Inter. Med. 1.996, 125 (11), 873-882 ] have been used for the treatment of cachexia in AIDS patients..

The relationships between nutrition, HIV infection and immune system [J.M. Hoyt et al., II. Assoc. Nurses AIDS care 1.991, 2 (3), 16-28,] and the role of certain cytokines in the wasting syndrome in AIDS patients, can be considered as the basis for new treatments involving the use of inmunomodulators. The use of thalidomide , an immunomodulating drug, in the treatment of the wasting syndrome associated to AIDS has recently been published [G. Reyes-Teran et al. , AIDS, 1.996, 10, 1501-1507].

On the other hand, cachexia is also associated to the high mortality rate in cancer patients, this rate being estimated by some authors as 30-70% . Many cancer patients die after continuous weight loss and generalized weakness,. Oncology patients with associated cachexia have a negative prognostic as the possibilities of effective chemoterapeutic treatments are substantially reduced. Consequently, an effective treatment of cachexia should improve the general condition of the patient : more effective chemotherapy, better quality of life and more favorable prognosis of the disease could be achieved.

However, all the approaches for slowing down or reverting cachexia have been focused on the improvement of nutritional supply through special diets and regimen without positive results.

Recent publications are now stressing the relationships between the high levels of certain pro-inflammatory citokynes and cancer cachexia: TNFα, IL-1, IL-6 and IFN gamma [P.Elliot et al., Drug &. Market Development 1.98, 9, 26-28 .] This dysfunction of the immune system, together with reduction in the NK cell activity and plasma levels of cystine and glutamine, accompanied of urea overproduction, shown in different pathologies, is extremely important in the induction and progression of cachexia in cancer patients. Although the induction mechanism by such cytokines is unknown, antagonizing their effects by using inmunomodulators is one of the most updated approaches to revert cachexia.

It is obviously necessary to develop new ways to stop or revert the cachetic syndrome in order to improve the quality of life and to enhance the efficacy of basic treatments.

The fern known for years as Polypodium leucotomos has historically been used by the Honduran natives, as infusions of leaves and rhizomes, for the treatment of malign tumors, rheumatoid arthritis and psoriasis. Horvath et al. [ Nature, 1967, 214 , 1256- 1258 ] showed both the in vitro and in vivo antitumor effect of an aqueous extract of the fern. Later, a water soluble fraction from the leaves of Polypodium leucotomos has been widely studied . Its inmunomodulator / inmunosuppresor profile and its antioxidant and anti-free radical properties have been described [M.D. Fernandez & al. 1^{st} World Congress on Medicinal and Aromatic Plant for Human Welfare, Maastricht, 1.992; J. Rayward et al., 2^{nd} International Congress on Biological Response Modifiers, San Diego, USA; S. Gonzalez & al., Photodermatol Photoimmunol, Photomed, 1.996,12,45 ].

The use of extracts from the genus Polypodium, and more specifically from Polypodium leucotomos, as antioxidants and photoprotectants has been claimed in the patent PCT/US 96/01808 by Pathak et al. (1996).

The present invention provides a water soluble fraction obtained from the leaves of a cultivated variety of Phlebodium decumanum, purified and standardized, identified as EXPLY-37, its production and its use in the manufacturing of formulations useful as nutritional supplements.

### DESCRIPTION OF THE INVENTION

This invention is addressed, but not limited, to the manufacturing of a water-soluble fraction from the leaves of a cultivated variety of Phlebodium decumanum, purified and standardized, identified as EXPLY-37, to the preparation of EXPLY- 37 formulations and to the use of these formulations as nutritional supplements in AIDS and cancer patients. The formulations can be used together with anti-retroviral drugs in AIDS patients and associated to conventional oncology treatments (surgery and /or radio-and chemotherapy), in order to improve the efficacy and reduce the side effects in the recovery of patients under those treatments.

### 1. Phlebodium decumanum

This Polypodiacee, belonging to the Subgenus Phlebodium, within the Genus Polypodium, is organically cultivated and processed in the facility near the Yojoa lake (Northern Honduras). This facility is owned by HELSINT S.A.L. (Spain) and exploited by Helechos International, Honduras, S.A. (HIH) . The plant has been considered for years as Polypodium leucotomos but has been reclassified in 1,992 according to the in situ studies carried out by Prof.Cirile Nelson, Director of the Herbario TEFH , Department of Biology, UNAH ( Tegucigalpa, Honduras ), Prof. Sinn Sandberg, Uppsala University, and Prof. Antonio Molina, from the Paul C. Stanley Herbario and the Escuela Agricola Panamericana. A full agreement has been reached on the new nomenclature. The relationships among the different Polypodium species have been furthermore reviewed by M. Väsange, Ph. Thesis, Uppsala University, 1,996..

The lake Yojoa cultures are the only ones existing in the world where both ferns - Phlebodium decumanum and Polipodium leucotomos -, are cultivated.

### 2. Water-soluble fractions of Phlebodium -decumanum.

EXPLY-37 is the tradename, property of HELSINT S.A.L. and applied to the **E**xtract of **P**hlebodium decumanum from the Yojoa Lake. N^{o} 37 corresponds to the extraction method selected among those tested for the preparation of the extract from the leaves and is based on the methods disclosed in patents G B 2.024. 622 A, GB 2075.834-A, ES 8902092 and more recently in WO 96/5139.

Sporulated leaves from Phlebodium decumanum, dried and ground, are delipidated by treatment with petroleum ether, methylene choride or mixtures petroleum ether/methylene chloride, then extracted with a mixture of methanol/water. An alternative method consists in the extraction with a mixture of methyl alcohol / water followed by delipidation of the extract with petroleum ether, methylene chloride or mixtures petroleum ether / methylene chloride.

By removing the methyl alcohol at reduced pressure the remaining water-soluble fraction is purified by passing it through a mixed ion-exchange column, treatment with active charcoal, sodium metabisulphyte and filtration.

The purified water-soluble fraction is concentrated until a batch-to-batch constant and reproducible composition within a narrow range. Example 1 describes a method for The preparation of a water-soluble fraction from the leaves of Phlebodium decumanum identified as EXPLY-37. A typical composition of EXPLY-37 is shown in Table 1.

The tradename EXPLY-37 has been applied by HELSINT S:A:L to distinguish the extract from the variety of Phlebodium decumanum cultivated in the lake Yojoa facility and to differentiate it from wild varieties of Phlebodium decumanum from other origins.

A water-soluble fraction, purified and standardized, can also be obtained from the rhizome by the following method: the rhizome, after removing all the villosities, is washed with an sterilizing solution and deionized water, dried, ground, and extracted with a mixture of methyl alcohol/water. After removal of the alcohol in the vacuum, the cloudy water-soluble fraction is treated with petroleum ether, methylene chloride or mixtures petroleum ether/methylene chloride to remove the remain lipids. The solution is clarified with active charcoal, treated with sodium metabisulphite, filtered and concentrated until a batch-to-batch constant and reproducible composition, within a narrow range. Example 2 describes a method for the preparation of a water-soluble fraction, or extract, from the rhizome of Phlebodium decumanum. A typical composition of such fraction is shown on TABLE 2.

### 3. Formulations containing EXPLY-37 for use by oral route

The invention provides formulations containing EXPLY-37 useful as nutritional supplements. These formulations may additionally contain rhizome and/or rhizome water-soluble fraction. The formulations, adequate for oral administration, can be solid or liquid and used as powders, soft or hard gelatine capsules or syrups.

### 3.1 Solid formulations containing EXPLY-37

### 3.1.a) Mixture containing powdered rhizome of Phlebodiun decumanun and EXPLY-37.

This preparation can be obtained by a method consisting of the following steps:
- successive washings of the rhizome with water containing active chlorine, deionized water, ethyl alcohol and de-ionized water.
- drying of the rhizome • grinding of the rhizome and homegenization to a selected particle size
- incorporation of EXPLY-37
- drying.

This mixture of EXPLY-37 and Phlebodium decumanum rhizome, ground and homogenized, can be used as a powder to fill hard gelatine capsules containing between 100 and 500mg powder. The ratio [ground and homogenized rhizome: EXPLY-37 ] in the mixture is within the range 4:1 to 1:1.

### 3.1.b) Mixture containing powdered Phlebodium decumanum rhizomes. EXPLY-37 and Phlebodium decumanun rhizome extract.

This preparation can be obtained by a similar method to that summarised in 3.1 .a), although different mixtures of EXPLY-37 and rhizome extract (50:50 to 95:5) can be added in different proportions. The solid mixture containing EXPLY-37, rhizome powder and rhizome extract of Phlebodium decumanum, prepared as a dry powder, can be used to fill hard gelatine capsules containing 100-500 mg solid powder. The relative proportions [EXPLY-37 + rhizome extract ] : [ rhizome ] can be in the range 4:1 to 1:1

### 3.2 Liquid formulations containing EXPLY-37

These formulations can be prepared as soft gelatine capsules and syrups.

### 3.2.a) Soft gelatine capsules containing EXPLY-37

The capsules con be filled by direct injection of diluted EXPLY-37 having the adequate viscosity. These capsules can contain 50-700mg EXPLY-37.

### 3.2.b) Soft gelatine capsules containing EXPLY- 37 and Phlebodium decumanum rhizome extract.

These capsules can be obtained by the following process:
- Preparation of a homogeneous mixture containing EXPLY-37 and rhizome extract, the relative proportions ranging within the range ( 50: 50 to 95 : 5)
- Dilution of the mixture and injection in soft gelatine capsules. Total content [EXPLY-37 + rhizome extract] : 50 to 750mg:

### 3.2.c) Syrups containing EXPLY-37

A syrup can be prepared by homogenizing EXPLY-37 with the following components:
- Saccharum officinarum liquid extract
- Glycyrrhiza glabra liquid extract
- Citric acid

Preferred formulations are those containing EXPLY-37 at a concentration of 50-500mg/g.

### 3.2. d) Syrups containing mixtures of EXPLY-37 + Phlebodium decumanum rhizome extract [relative proportions (50 : 50) to (95 :1)] and the following components:

- Sacharum officinarum liquid extract
- Glycyrrhiza glabra liquid extract
- Citric acid

Preferred formulations are those containing the mixture [EXPLY-37+rhizome extract] at a concentration of 50-500 mg/g.

### 3.2.e) Syrups containing EXPLY-37.

A different syrup can be prepared by the incorporation of EXPLY-37 to an aqueous solution containing the following excipients:
- invert sugar
- sorbitol
- propylenglycol

Preferred formulations are those containing EXPLY-37 at a concentration of 20-500 mg /ml.

### 3.2.f) Syrups containing EXPLY-37 and Phlebodium decumanum rhizome extract.

Syrups can be prepared by incorporating a mixture containing various proportions [EXPLY-37 : rhizome extract (50: 50) to (95 : 5)] into an aqueous solution of following excipients:
- invert sugar
- sorbitol
- propylenglycol

Preferred formulations are those containing 20-50 mg [EXPLY-37 + rhizome extract / ml ]

EXPLY-37 formulations provided in this invention are useful as nutritional supplement of general use.

These formulations are, particularly adequate for the treatment of malnutrition, body weight loss, generalized wasting and cachectic syndrome in AIDS patients [ see the results obtained with AIDS patients: Clinical Results. I. AIDS patients with advanced HIV infection] and as a supplement to treatments with anti-retroviral drugs. The formulations are also useful for the treatment of cancer patients showing generalized weakness and cachectic impairment : as cachexia is slowing- down and reverting, a general improvement is obtained allowing a more efficacious application of the appropriate anti-cancer treatments. EXPLY-37 formulations can also be used as adjuvant in conventional oncology treatments (surgery and/or radio-and chemotherapy ) and for the recovery of cancer patients, previously under such treatments [ see the results obtained in cancer patients : Clinical Results. II. Treatment of cancer patients ].

Treatment of cachectic syndrome in patients with advanced HIV infection is better performed by administration of the formulations provided in this invention at daily doses comprised between 1 and 5 g, expressed as EXPLY-37. Similar doses are adequate for the treatment of cachectic syndrome in cancer patients, as adjuvant in conventional anti-cancer treatments and in the recovery of cancer patients following to those treatments.

The following examples illustrate well the invention although they must not be considered limiting of the same.

### EXAMPLE 1

### Preparation of EXPLY-37 from Phlebodium decumanum leaves.

Sporulated, freshly, harvested leaves from Phlebodium decumanum are dried for 1 week in a drying room by introducing pressed air at 40-50° C . Once dried, the leaves are ground. A mixture of small particles and fine powder is obtained. 100kg dry, ground leaves are extracted 3 times with 400 liners of 7/3 methyl alcohol/de-ionized water at 40°C for 12 hours. Hydromethanolic extracts are collected and concentrated at 40° C under reduced pressure until complete removal of the alcohol. The fine suspension obtained is treated 3 times with an equal volume of non-polar solvent, preferently petroleum ether or petroleum ether/ dichoro methane mixtures, in order to remove any residual lipids. The delipidated water soluble fraction is purified by passing it through an ion-exchange resin column followed by treatment with active charcoal, sodium metabisulfite and filtration. Concentration of the solution under reduced pressure, until a final waxer content of 20%, yields 5-10% (5-10kg) of EXPLY-37.

**Table 1**

| **Composition of the purified, standardized water-soluble fraction from Phlebodium decumanum leaves (EXPLY-37).** | | |
|---|---|---|
| | | % |
| Total solid content | | 79,5- 81,5 |
| Ash | | 10-12 |
| Total N | | 0,7- 0,9 |
| Total protein | | 4,9- 5,9 |
| Lipids | | < 0,2 |
| Carbohydrates | | 60,5- 64,5 |
| pH | 5-5,1 | |
| Refractive index | 1,4- 1,5 | |
| UV absorption | 235- 237 nm | |
| | 274- 284 nm | |
| Absorbance at 290 nm | 2,5-5 | |

### EXAMPLE 2

### Preparation of extracts from Phlebodium decumanun rhizome

100 kg wet rhizomes, freshly harvested are successively extracted at 40° C, for 10 hours with:
a) 400 L methylalcohol
b) 400 L methylalcohol / water ( 8:2)
c) 400 L methylalcohol / water ( 7 : 3)

The different extracts are combined and concentrated at 40° C and 25 mm until complete removal of the alcohol. The cloudy aqueous solution is extracted twice withmethylene chloride or mixtures methylene chloride: petroleum ether.

The aqueous phase is then treated with active charcoal until a final absorbance of 0,5- 1,5 at 290 nm is reached. Final concentration of the solution at 40° C and 25 mm pressure gives a water-soluble fraction with a total solid content of 80%. A yield of 1- 3 kg of purified and standardized rhizome extract is obtained. Its composition is batch to bath constant and reproducible within narrow limits (Table 2).

**Table 2**

| **Composition of the purifieds standardized water-soluble fraction from Phlebodium decumanum rhizomes** | | |
|---|---|---|
| | | % |
| Total solid content | | 80- 81 |
| Ash | | 10-12 |
| Total N | | 0,6-0,7 |
| Total protein | | 4- 4, 5 |
| Lipids | | 0,5 |
| Carbohydrates | | 65- 66 |
| pH | 5-5,1 | |
| Refractive index | 1,4- 1,5 | |
| UV absorption peaks at | 237 nm and 272 nm | |
| Absorbance at 290 nm | 0,5- 1,5 | |

### EXAMPLE 3

### Capsules containing EXPLY-37 and rhizome

50kg fresh rhizomes from Phebodium decumanum, freed from villosities, are washed by successive immersions in a dilute aqueous solution containing active chlorine, de-ionized water, absolute ethyl alcohol and sterile de-ionized water. The rhizomes are dried in a fluid layer dryer at 40°-50° C for 2 hours. The dry rhizomes are ground and sieved. A fine powder is obtained.

7,5 Kg EXPLY- 37 are added to 22,5 kg rhizome powder with stirring in a 100 L capacity stainless steel mixer. Stirring continues until a homogeneous mixture is obtained. The mixture is then dried on plates in the oven at 40- 50° C for 24 hours. The powder is sieved through a 18 mesh sieve. Capsules containing 300 mg powder are prepared.

### EXAMPLE 4

### Capsules containing EXPLY- 37 enriched powder

By following a process similar to that described in Example 3, the mixture is prepared with 25,5 Kg rhizome powder and 18, 5 Kg EXPLY- 37. After drying homogenizing and sieving, capsules containing 440 mg powder are prepared.

### EXAMPLE 5

### Capsulas containing EXPLY- 37, rhizome and rhizome extract.

By following a similar process to that described in Example 3, a final mixture containing 26,8 kg powdered dry rhizome, 10 kg EXPLY- 37 and 3,2 kg rhizome extract is prepared. Capsules containing 400 mg dry powder are manufactured . The relative proportions [XPLY-37 : rhizome extract] can vary within a wide range ( 50 : 50) to (95 : 5)

### EXAMPLE 6

### EXPLY- 37 syrup

A homogeneous mixture is made up of 7,8 kg of Saccharum officinarum liquid extract and 190g glycyrrhiza glabra liquid extract by stirring in a stainless steel mixer tank provided of a paddle stirring element at 20 rpm. 10g citric acid are then added, followed by 2kg EXPLY- 37. Stirring is continued at 37° C until complete homogenization. A syrup containing 200 mg EXPLY- 37 /g is obtained.

By using different amounts of EXPLY- 37 and making the necessary corrections in the amounts of excipients, syrups containing 50- 500 mg EXPLY-37/g are prepared.

### EXAMPLE 7

### Syrups containing EXPLY- 37 and rhizome extract.

By following the steps described in Example 6, a syrup containing 1 kg EXPLY- 37 + 1 kg rhizome extract is obtained. Final concentration : 200mg whole extract/g.

Syrup having a composition in the range 50- 500 mg total extract/g can be prepared in a similar way. The relative proportions [EXPLY-37 : rhizome extract] can vary from (50 :50) to (95 : 5)

### EXAMPLE 8

### Syrups containing EXPLY- 37 and rhizome extract.

5,000 g invert sugar, 2,500 g sorbitol and 50 g propylenglycol are successively added on 2,450 mL de-ionized water in a glass tank provided of anchor stirring element. When the mixture is fully homogenized, 2.5 Kg EXPLY-37 or 2 Kg EXPLY-37 and 0.5 Kg rhizome extract are added under slow stirring at temperature of 30°-40° C. Syrup concentration : 200 mg / ml, expressed as EXPLY-37 or EXPLY-37 + rhizome extract].

### CLINICAL RESULTS

### I. Patients with advanced HIV infection

The number of HIV infected patients is reaching dramatic levels in Central America and Caribbean counties. Honduras' case is known by the number of patients in advanced stager of the disease, the early age of many of these patients and the lack of resources for diagnosis, prevention and treatment of the disease with anti-retroviral drugs.

Honduran patients, knowing the existence of the lake Yojoa Polypodiacee cultures, requested some compassionate remedies for the improvement of their general condition. HIH decided to prepare different formulations to be supplied as nutritional and energetic supplements to those patients willing to use the remedy. Said formulations are within the scope of this patent application

### 1. Dose exploration in individual patients

Preliminary results correspond to five advanced patients in very deteriorated conditions. No inclusion criteria were applied. These patients were given, together with the regular diet, EXPLY-37 syrup containing only EXPLY-37 and prepared as in Example 8. Daily doses were given thrice daily and ranged between 1 and 3 g / day. After the first month of treatment all five patients received 3 g / day. Results are summarised in table 3

**Table 3**

| **Results in isolated cases** | | | | |
|---|---|---|---|---|
| **Patient** | **Age/sex** | **Appetite** | **Months of treatment** | **Body weight Quality of life** |
| 1 | 52/M | 5+ | 9 | Body weight increase to normal figures. |
| | | | | Professional activity became to normal at the 2^{nd} month |
| 2 | 21/F | 4+ | 5 | Body weight increase to normal figures. Quality of life |
| 3 | 22/M | 4+ | 5 | Body weight increase to normal figures. Quality of life |
| 4 | 27/M | 3+ | 4 | 60% body- weight increase. |
| | | | | Professional activity became to normal at the 1^{st} month of treatment |
| 5* | 20/M | - | - | |

| | | | | |
|---|---|---|---|---|
| *This patient died two days after initiating the treatment. Appetite scale 1+ to 5+. | | | | |

### 2. Groups of controlled patients

A first group of 5 patients was treated according to a simple protocol including the following criteria:
- Age: 18- 35 years
- Number of patients: 10, both sexes

### Inclusion criteria

Advanced HIV infection
Absence of opportunistic infection
No treatment with anti-retroviral drugs
CD4+ + not lower than 400
Body weight loss in the previous 6-month period not lower than 10%

### Parameters to be measured

Appetite recovery
Body weight evolution
Quality of life

This group of patients is on the 2^{nd} month of treatment. They are receiving as a nutritional supplement 3,1g EXPLY- 37 doses par day as a syrup prepared according to Example 8. Table 4 summarizer the results corresponding to the first 5 controlled patients.

**Table 4**

| **Results in groups of controlled patients** | | | | |
|---|---|---|---|---|
| **Patient** | **Age/sex** | **Appetite** | **Months of treatment** | **Body weight Quality of life** |
| 1 | 32/M | 3+ | 2 | Progressive body weight gain. |
| | | | | Improvement in the quality of life |
| 2 | 33/F | 3+ | 2 | Id |
| 3 | 26/F | 3+ | 2 | Id |
| 4 | 27/M | 4+ | 2 | Id |
| 5 | 33/F | 3+ | 2 | Id |

The initial inclusion criteria were modified from patient n^{o}6 on due to the difficulties in selecting patients with CD4+ count not lower than 400 : most of the examined patients showed a CD4 + count between 100 and 200 and were suffering from different associated opportunistic infections.

A second group of patients meeting the new inclusion criteria are being treated with capsules containing [EXPLY- 37 +rhizome] and prepared as in Example 4 . The daily dose is 6-12 capsules /day. The first 4 patients showed a general improvement similar to that observed in the previous groups : rapid appetite gain, smooth and progressive body-weight gain, improvement in the quality of life and resumption of both personal and professional activities in those cases where these activities had been discontinued.

### II. Treatment of cancer patients

Continuous use of different EXPLY-37 formulations in cancer patients with different type of tumors at different stages, either during conventional anticancer treatments or following to the application of such treatments has shown the efficacy of said formulations in:
- reversion of cachexia and quality of life recovery in very ill patients
- application of new chemotherapy protocols in recovered patients.
- maintenance of good general condition and quality of life in said patients.
- reduction of the undesirable effects in patients under chemotherapy.
- general condition and quality of life recovery following to the application of conventional anticancer treatments.

Experience has been accumulated for several years after the positive results obtained with the first patient.

Table 5 summarizes some representative cases where a relationship between the positive evolution in the patient condition is associated to the continuous use of EXPLY-37.

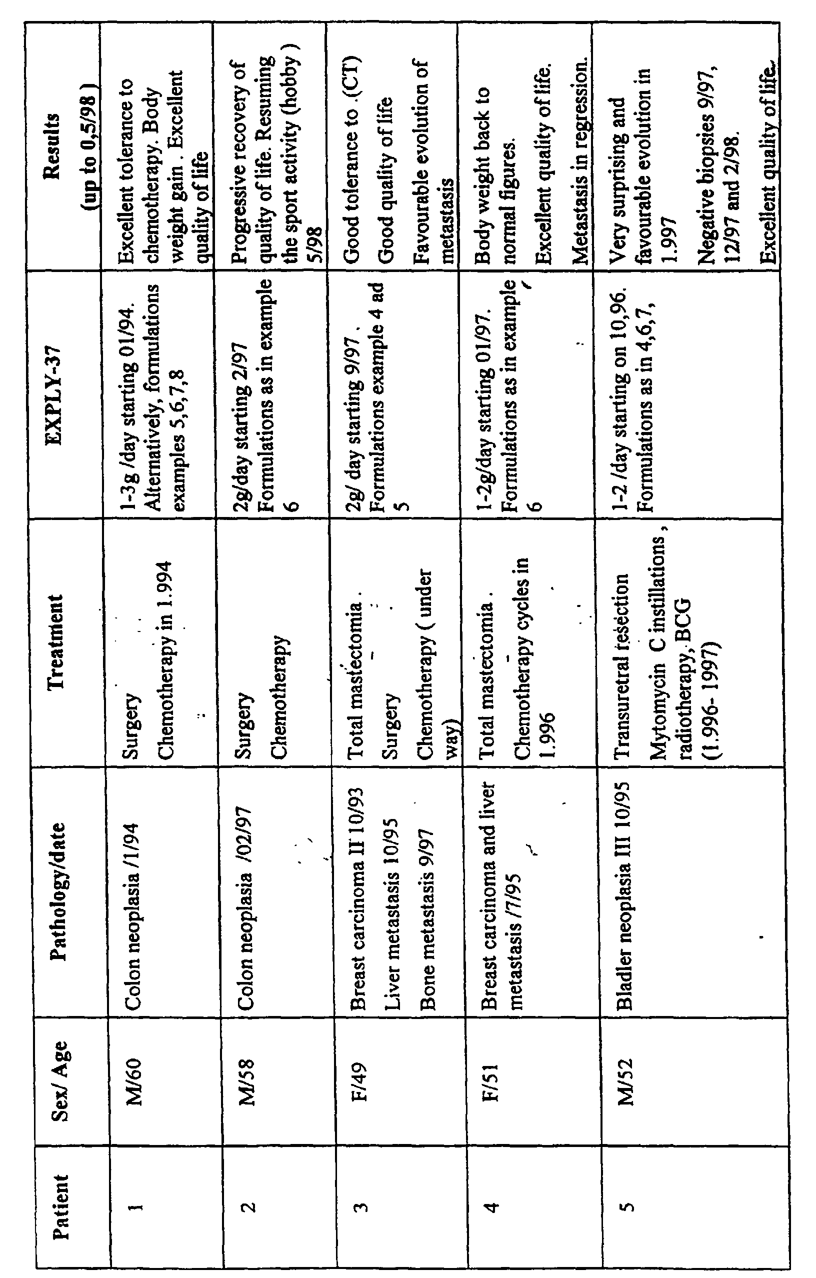

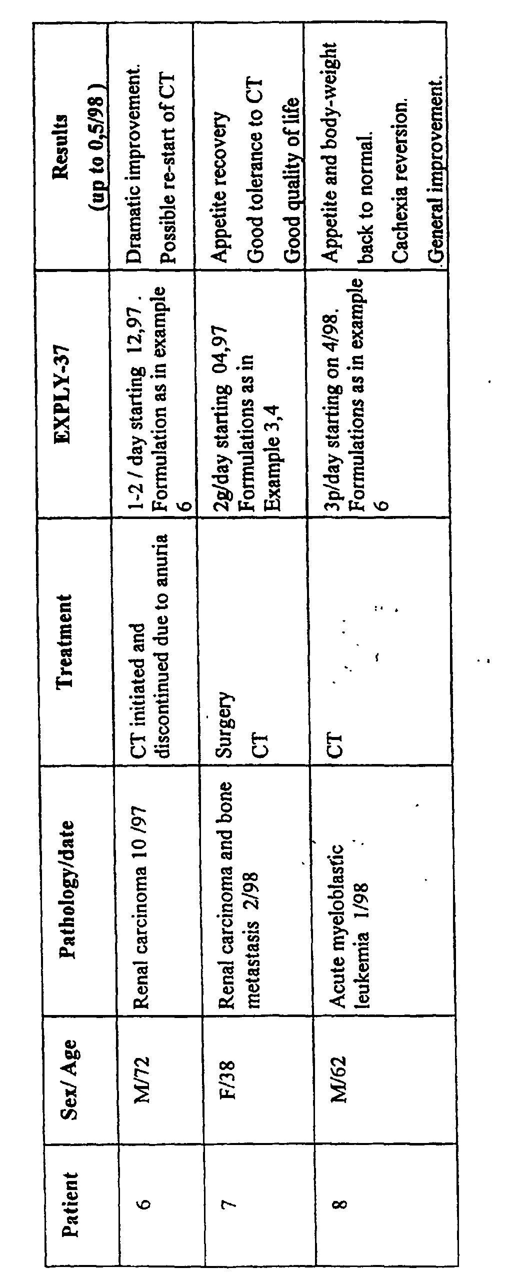

## Claims

1. A purified and standardized water-soluble fraction from the leaves of the variety of Phlebodium decumanum organically cultivated in the lake Yojoa facility (Northern Honduras), identified as EXPLY-37 and characterized by the following specifications:
| | | |
|---|---|---|
| | | % |
| Total solid content | | 79,5 - 81,5 |
| Ash | | 10 - 12 |
| Total N | | 0,7 - 0,9 |
| Total protein | | 4,9 - 5,9 |
| Lipids | | < 0,2 |
| Carbohydrates | | 60,5 - 64,5 |
| pH | 5- 5,1 | |
| Refractive index | 1,4- 1,5 | |
| UV absorption | 235- 237 nm | |
| | 274- 284 nm | |
| Absorbance at 290 nm | 2,5-5 | |

2. A method for the preparation of a purified and standardized water-soluble fraction from the leaves of the variety of Phlebodium decumanum grown organically cultivated in the lake Yojoa facility (Northern Honduras), identified as EXPLY-37, according to claim 1, comprising the following steps:
a) lipid removal from the dry, ground, sporulated Phlebodium decumanun leaves, by treatment with petroleum ether, methylene chloride., or mixtures petroleum ether/methylene chloride
b) extraction of the water-soluble fraction with a mixture methyl alcohol/water.
c) Methyl alcohol removal at reduced pressure
d) Purification of the water-soluble fraction obtained in c) by passing the solution through a mixed ion-exchange column, treatment with active charcoal, sodium metabisulphite and filtration
e) Concentration of the purified, water-soluble fraction, until a batch-to-batch constant and reproducible composition.

3. A method for the preparation of a purified and standardized water-soluble fraction from the leaves of the variety of Phlebodium decumanum organically cultivate in the Yojoa lake facility (Northern Honduras), identified as EXPLY-37, according to claim 1, comprising the following steps:
a) extraction of the dry, ground, sporulated leaves of Phlebodium decumanum with a mixture methyl alcohol/water
b) methyl alcohol removal at reduced pressure
c) lipid removal of the solution obtained in b) by extraction with petroleum ether, methylene chloride or mixtures petroleum ether/methylene chloride
d) purification of the water-soluble fraction obtained in c) by passing the solution through a mixed, ion-exchange column, treatment with active charcoal, sodium metabisulphite and filtration
e) concentration of the purified water-soluble fraction until a batch to batch constant and reproductible composition.

4. Formulations comprising a purified and standardized water soluble fraction from the leaves of the variety of Phlebodium decumanum organically cultivated in the Yojoa lake facility (Northern Honduras), identify as EXPLY-37, according to claim 1 and obtained according to the method described in claims 2 or 3.

5. EXPLY-37 formulations, according to claim 4, containing 50-750 mg EXPLY-37 in soft gelatine capsules.

6. Formulations, according to claim 4, containing EXPLY-37 and extract of Phlebodium decumanum rhizome. These formulations are prepared as soft gelatine capsules containing 50-750mg of the mixture of [EXPLY-37 + rhizome extract].

7. Formulations, according to claim 6, wherein the relative proportions EXPLY-37: rhizome extract can be within the range (50:50) to (95:5).

8. Formulations, according to claim 4, containing a dry powder prepared with EXPLY-37 and ground, homogenised Phlebodium decumanum rhizome. The formulations are prepared as hard gelatine capsules containing 100-500 mg powder.

9. Formulations, according to claim 8, wherein the w/w ratio (rhizome:EXPLY-37) is comprised between (4:1) and (1:1).

10. Formulations, according to claim 4, containing a dry powder prepared with EXPLY-37, Phlebodium decumanum rhizome, extract and ground, homogenized rhizome, These formulations are delivered as hard gelatine capsules containing 100-500 mg powder.

11. Formulations, according to claim 10, wherein the relative proportions [EXPLY-37: rhizome extract] are within the range (50:50) to (95:5).

12. Formulations, according to claim 10, wherein the w/w ratio [EXPLY-37 + rhizome extract: ground homogenized rhizome] can be (4:1) to (1:1).

13. Formulations, according to claim 4, containing EXPLY-37 together with the adequate excipients for liquid preparations.

14. Formulations, according to claim 13, wherein excipients are selected among Saccaharum officinarum liquid extract, Glycyrrhiza glabra liquid extract, citric acid and mixtures of them and are prepared as syrups having a concentration of 50-500 mg EXPY-37/g.

15. Formulations, according to claim 4, containing EXPLY-37 + Phlebodium decumanum rhizome extract, Saccharum officinarum liquid extract Glycyrrhiza glabra liquid extract, citric add and mixture of them, and are prepared as Syrups having a concentration of 50-500 mg (EXPLY-37 + rhizome extract)/g.

16. Formulations, according to claim 15, wherein the ratio [EXPLY-37: rhizome extract] is in the range 50:50-95:5.

17. Formulations, according to claim 13, wherein the excipients are selected among inverted sugar, sorbitol, propylenglycol and mixtures of them, and are used as Syrups containing a concentration of 20-500 mg/ml expressed as EXPLY-37.

18. Formulation, according to claim 4, containing Exply-37 + Phlebodium decumanum rhizome extract and excipients selected among ininvert sugar, sorbitol, propylenglicol and mixtures of them, and conditioned as syrups having a concentration of 20-500mg/ml expressed as [EXPLY-37 + rhizome extract].

19. Formulations, according to claim 18, wherein the ratio EXPLY-37: rhizome extract is in the range (50:50)-(95:1).

20. Nutritional supplement comprising any of the formulations claimed in claims 4 to 19.

21. Use of a nutritional supplement as in claim 20 for treating cachexia and wasting syndrome in AIDS patients.

22. Use of a nutritional supplement as in claim 20 in AIDS patients treated with antiretroviral drugs.

23. Use of a nutritional supplement as in claim 20 for treating cachexia and wasting syndrome in cancer patients.

24. Use of a nutritional supplement as in claim 20 for treating cancer patients under treatment with conventional oncologic therapies or as a follow up to those treatments (surgery and/or chemotherapy and radiotherapy).
